# EUROPEAN PATENT APPLICATION

(11) **EP 3 372 162 A1**
(43) Date of publication of application: **12.09.2018**
(21) Application number: 17160294.9
(22) Date of filing: 10.03.2017
(51) Int. Cl.: A61B 5/11, G08B 21/04, A61B 5/00, G08B 13/196

(54) **A METHOD, APPARATUS AND SYSTEM FOR MONITORING A SUBJECT IN AN ENVIRONMENT OF INTEREST**

(71) Applicant: Koninklijke Philips N.V., 5656 AE Eindhoven (NL)
(72) Inventor: TEN KATE, Warner Rudolph Theophile, 5656 AE Eindhoven (NL); VAN DE WOUW, Doortje, 5656 AE Eindhoven (NL)
(74) Representative: de Haan, Poul Erik

(57) **Abstract**

There is provided a method for monitoring a first subject in an environment of interest using a first movement sensor for measuring movements of the first subject, and a first motion sensor in the environment of interest for measuring motion in a first sensing region, the method comprising obtaining measurements of the movements of the first subject over time from the first movement sensor; obtaining measurements of the motion in the first sensing region from the first motion sensor; and determining if motion measured by the first motion sensor is motion by the first subject by comparing the measurements of the movements and the measurements of the motion.

## Description

### Technical Field

The invention relates to the monitoring of a subject in an environment, and in particular in an environment that includes one or more motion sensors.

### Background of the Invention

Providing at-home elderly care services is a fast growing market which helps elderly people to live independently in the comfort of their home, while still having access to medical support when needed. These services typically include a home monitoring system that enables the provision of remote care, removing the need for the person to go to, or to transfer to a care facility. Those home monitoring systems may report the activities of daily life (ADLs), also known as the activities of daily living, that the person is, has or has not been performing. These activities can include when the person is sleeping, going for a walk (and where), when the person uses a toilet, the room of the home that the subject is in, etc.

Some monitoring systems make use of wall- or ceiling- mounted sensors, such as passive infrared sensors (PIRs), that can monitor a sensing area, or a sensing region. These sensors sense movement in the sensing region, and they can be triggered by movement of an object, a person or an animal (e.g. a dog or cat). Thus, one of the problems associated with these monitoring systems is distinguishing between the activities of interest (i.e. those by the person receiving care) and those by other people or animals present in the house. For example this can include partners or spouses living in the same household or visitors (e.g. nurse, social worker, care giver, etc.), but can include pets. In the United States it is understood that between 40-60% of elderly people who live alone and require home monitoring have a cat or a dog.

### Summary of the Invention

Therefore there is a need for an improved method, apparatus and system for monitoring a subject in an environment of interest, for example in a living environment, such as a house, a room, an apartment, etc.

According to a first aspect, there is provided a method for monitoring a first subject in an environment of interest using a first movement sensor for measuring movements of the first subject, and a first motion sensor in the environment of interest for measuring motion in a first sensing region, the method comprising obtaining measurements of the movements of the first subject over time from the first movement sensor; obtaining measurements of the motion in the first sensing region from the first motion sensor; and determining if motion measured by the first motion sensor is motion by the first subject by comparing the measurements of the movements and the measurements of the motion. This method provides a simple approach to determining whether motion sensed by a motion sensor is motion by the first subject based on corresponding movement of the first subject measured by a movement sensor, and thus does not require any complex processing of the measurements or the use of a dedicated location system.

In some embodiments, the step of determining comprises determining that motion measured by the first motion sensor is motion by the first subject and that the first subject is in the first sensing region if the motion measured by the first motion sensor occurs during a time period in which the measurements of the movements indicate that the first subject is moving. In some embodiments, the step of determining comprises determining that motion measured by the first motion sensor is not motion by the first subject if the motion measured by the first motion sensor occurs during a time period in which the measurements of the movements indicate that the first subject is not moving. Thus, in both of the above embodiments, only simple processing is required in order to determine whether motion sensed by a motion sensor is motion by the first subject.

In some embodiments, the method further comprises the step of obtaining measurements of motion in a second sensing region from a second motion sensor; and the step of determining comprises determining if motion measured by the first motion sensor and/or motion measured by the second motion sensor is motion by the first subject by comparing the measurements of the movements, the measurements of the motion from the first motion sensor and the measurements of the motion from the second motion sensor. Thus, these embodiments provide the advantage that motion of the first subject can be identified in several different regions.

In the above embodiment, the step of determining if motion measured by the first motion sensor and/or motion measured by the second motion sensor is motion by the first subject can comprise determining that motion measured by the first motion sensor is motion by the first subject if the motion measured by the first motion sensor occurs during a time period in which the measurements of the movements indicate that the first subject is moving; and/or determining that motion measured by the second motion sensor is motion by the first subject if the motion measured by the second motion sensor occurs during a time period in which the measurements of the movements indicate that the first subject is moving.

In some embodiments, a second subject has a second movement sensor for measuring the movement of the second subject, the method further comprises the step of obtaining measurements of the movements of the second subject from the second movement sensor; and the step of determining comprises determining if motion measured by the first motion sensor is motion by the first subject or the second subject by comparing the measurements of the movements of the first subject, the measurements of the movements of the second subject and the measurements of the motion. These embodiments provide the advantage that it is possible to determine whether detected motion is motion of a first subject or motion of a second subject, such as another person or a pet.

In the above embodiment, the step of determining if motion measured by the first motion sensor is motion by the first subject or the second subject can comprise determining that motion measured by the first motion sensor is motion by the first subject if the motion measured by the first motion sensor occurs during a time period in which the measurements of the movements indicate that the first subject is moving; and determining that motion measured by the first motion sensor is motion by the second subject if the motion measured by the first motion sensor occurs during a time period in which the measurements of the movements indicate that the second subject is moving.

According to a second aspect, there is provided a computer program product comprising a computer readable medium having computer readable code embodied therein, the computer readable code being configured such that, on execution by a suitable computer or processor, the computer or processor is caused to perform the method described above.

According to a third aspect, there is provided an apparatus for monitoring a first subject in an environment of interest, the apparatus comprising a processing unit configured to obtain measurements of movements of the first subject over time from a first movement sensor of the first subject; obtain measurements of motion in a first sensing region from a first motion sensor in the environment of interest; and determine if motion measured by the first motion sensor is motion by the first subject by comparing the measurements of the movements and the measurements of the motion. The apparatus provides a simple approach to determining whether motion sensed by a motion sensor is motion by the first subject based on corresponding movement of the first subject measured by a movement sensor, and thus does not require any complex processing of the measurements or the use of a dedicated location system.

In some embodiments, the processing unit is configured to determine that motion measured by the first motion sensor is motion by the first subject and that the first subject is in the first sensing region if the motion measured by the first motion sensor occurs during a time period in which the measurements of the movements indicate that the first subject is moving. In some embodiments, the processing unit is configured to determine that motion measured by the first motion sensor is not motion by the first subject if the motion measured by the first motion sensor occurs during a time period in which the measurements of the movements indicate that the first subject is not moving. Thus, in both of the above embodiments, only simple processing is required in order to determine whether motion sensed by a motion sensor is motion by the first subject.

In some embodiments, the processing unit is further configured to obtain measurements of motion in a second sensing region from a second motion sensor in the environment of interest; and the processing unit is configured to determine if motion measured by the first motion sensor and/or motion measured by the second motion sensor is motion by the first subject by comparing the measurements of the movements, the measurements of the motion from the first motion sensor and the measurements of the motion from the second motion sensor. Thus, these embodiments provide the advantage that motion of the first subject can be identified in several different regions.

In the above embodiment, the processing unit can be configured to determine that motion measured by the first motion sensor is motion by the first subject if the motion measured by the first motion sensor occurs during a time period in which the measurements of the movements indicate that the first subject is moving; and to determine that motion measured by the second motion sensor is motion by the first subject if the motion measured by the second motion sensor occurs during a time period in which the measurements of the movements indicate that the first subject is moving. These embodiments provide the advantage that it is possible to determine whether detected motion is motion of a first subject or motion of a second subject, such as another person or a pet.

In some embodiments, the processing unit is further configured to obtain measurements of movements of a second subject from a second movement sensor; and the processing unit is configured to determine if motion measured by the first motion sensor is motion by the first subject or the second subject by comparing the measurements of the movements of the first subject, the measurements of the movements of the second subject and the measurements of the motion.

According to a fourth aspect, there is provided a system for monitoring a first subject in an environment of interest, the system comprising an apparatus as described above; a first movement sensor for measuring the movements of the first subject; and an input for receiving measurements of motion in a first sensing region from a first motion sensor in the environment of interest.

In some embodiments, the system further comprises a first motion sensor for measuring motion in the first sensing region.

According to a fifth aspect, there is provided a system for monitoring a first subject in an environment of interest, the system comprising an apparatus as described above; a first motion sensor for measuring motion in a first sensing region; and an input for receiving measurements of movements of the first subject from a first movement sensor.

In some embodiments, the system further comprises a first movement sensor for measuring the movements of the first subject.

According to a sixth aspect, there is provided a system for monitoring a first subject in an environment of interest, the system comprising an apparatus as described above; a first movement sensor for measuring the movements of the first subject; and a first motion sensor for use measuring motion in the first sensing region in the environment of interest.

These and other aspects of the invention are apparent from and will be elucidated with reference to the embodiments described hereinafter.

It will be appreciated by those skilled in the art that two or more of the above-mentioned options, implementations, and/or aspects of the invention may be combined in anyway deemed useful.

### Brief Description of the Drawings

For a better understanding of the invention, and to show more clearly how it may be carried into effect, reference will now be made, by way of example only, to the accompanying drawings, in which:
Figure 1 is a block diagram of a system according to an embodiment;
Figure 2 is a diagram illustrating a system according to an embodiment in use in an environment of interest;
Figure 3 is a set of graphs illustrating measurements from three motion sensors in different rooms and two movement sensors on different subjects; and
Figure 4 is a flow chart illustrating a method of monitoring a subject according to an embodiment.

### Detailed Description of the Preferred Embodiments

As noted above, in a monitoring system that comprises one or more sensors that monitor respective sensing regions (otherwise referred to as sensing areas) in an environment of interest for motion, such as a home or care facility, it can be difficult to distinguish whether detected motion is motion of a subject of interest (i.e. a person receiving care), or motion by a different person or an animal. Conventional solutions provide that the subject of interest and/or other people and/or animals need to be tagged in some way and these tags tracked through the environment of interest. However, the invention provides a different approach, in which the subject of interest is provided with a sensor for directly sensing movements of the subject, and measurements from the movement sensor and motion sensor in the environment are compared to determine if motion measured by the motion sensor is motion by the subject of interest (and thus whether the subject is located in the sensing region of that motion sensor). This approach avoids the need for an additional or dedicated location system.

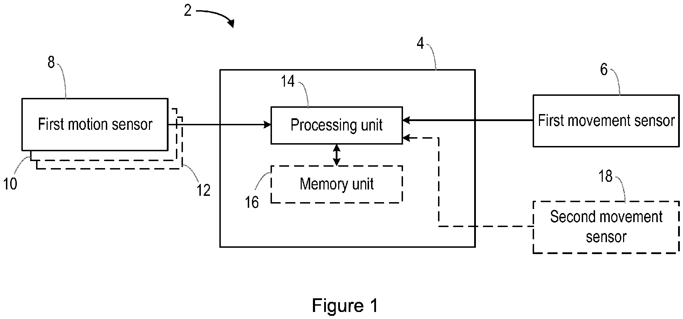

Figure 1 shows a system 2 for monitoring a subject in an environment of interest according to an embodiment, for example for monitoring the activities of daily living of a subject. The system 2 comprises three main components, an apparatus 4, a first movement sensor 6 that measures the movements of the subject to be monitored, and at least one motion sensor 8 that is for monitoring a region of the environment of interest. The environment of interest can be, for example, the home of the subject, a care facility where the subject lives, a hospital, etc.

The first movement sensor 6 is preferably worn or carried by the subject so that it can measure the movements of the subject. In some embodiments the first movement sensor 6 is an accelerometer that measures accelerations in three dimensions. The first movement sensor 6 can also or alternatively comprise an altitude sensor (e.g. an air pressure sensor) that measures the altitude or height of the subject, or changes in the altitude or height of the subject (for example to determine if the subject has gone or is going up or down the stairs). The first movement sensor 6 can also or alternatively comprise a position sensor for measuring the position of the subject, with movements of the subject being inferred by the measured position changing over time. The position sensor can be, for example, a satellite positioning system sensor, such as a GPS (Global Positioning System) receiver, that measures the location of the subject (and in some cases the speed of movement of the subject as well). The first movement sensor 6 can also or alternatively comprise a magnetometer, with movements of the subject being inferred from changes in the direction of the measured magnetic field. Those skilled in the art will be aware of other types of movement sensor that can be used in system 2. It will be appreciated that in some embodiments the first movement sensor 6 can provide measurements that enable an activity of the subject to be determined, e.g. walking, standing, running, going up or down stairs, etc., and references to measuring the movements of the subject should also be understood as referring to measuring or determining the activity of the subject.

In the case of the first movement sensor 6 being an accelerometer, the accelerometer can measure the magnitude of acceleration along three orthogonal axes (e.g. labelled X, Y and Z) and output three signals, each representing the magnitude of acceleration along a respective one of the axes, or output a single signal that is a composite of the accelerations measured along the three orthogonal axes. The accelerometer (or more generally the first movement sensor 6) can operate with any suitable sampling frequency, for example 50 Hertz (Hz), i.e. an accelerometer 6 can output an acceleration measurement every 1/50^{th} of a second, or for example 10 Hz. The output of the first movement sensor 6 represents measurements of movements of the subject over time.

In the case of the first movement sensor 6 being a magnetometer, the magnetometer can measure the magnitude of the magnetic field along three orthogonal axes and output three signals, each representing the magnitude of the magnetic field along a respective one of the axes, or output a single signal that is a composite of the magnetic field magnitudes measured along the three orthogonal axes.

In the case of an accelerometer or magnetometer, the variance of the magnitude along each axis can be measured and the components combined. The norm of the signals can also be computed as the Euclidean distance, which is also known as the L2 norm. Other methods are L1 norm (also known as the Manhattan distance), or max-min quartiles.

The at least one motion sensor 8 comprises a first motion sensor 8 that is for monitoring a region of the environment of interest, and in particular for measuring motion in a sensing region of the environment of interest. The at least one motion sensor 8 is to be placed in the environment of interest, for example attached or mounted to a wall or ceiling, or otherwise positioned in the environment, e.g. on a table. The motion sensor 8 can be any suitable type of sensor that can sense or measure motion in a sensing region. For example the motion sensor 8 can be a passive infrared (PIR) sensor. Alternatively the motion sensor 8 can be a sensor that detects when a door is opened and closed (and thus motion of a person can be inferred from the motion of the door). In another alternative the motion sensor 8 can be an accelerometer or pressure sensor attached to an object in the environment, e.g. a cup, plate, saucepan, etc. In another alternative, the motion sensor 8 can be a power sensor that detects when a subject has operated a device, such as a light switch or kettle. In another alternative, the motion sensor 8 can be a carbon dioxide sensor that measures changes in carbon dioxide levels consistent with a subject being present in the vicinity of the motion sensor 8. In another alternative, the motion sensor 8 can be a microphone or sound sensor that can measure the sound of a subject moving, e.g. walking. Those skilled in the art will be aware of other types of motion sensor 8 that can be used.

In a typical implementation, the system 2 comprises multiple motion sensors, and thus a first motion sensor 8 is shown in Figure 1, along with an optional second motion sensor 10 and optional third motion sensor 12. It will also be appreciated that a practical implementation of the invention can make use of multiple types of motion sensor 8 to monitor regions in the environment. It will be appreciated that where multiple motion sensors 8, 10, 12 are used, the motion sensors can be located or positioned in different rooms of, e.g. a house, apartment, hospital, etc. For example a first motion sensor 8 can be located in a bathroom to monitor motion in the bathroom, a second motion sensor 10 can be located in a bedroom to monitor motion in the bedroom, etc. It will also be appreciated that it is possible for multiple motion sensors to be located in the same room, for example where the room is quite large and it is not possible to monitor all parts of the room with a single motion sensor.

In some embodiments, the apparatus 4 is in a form that can be worn or carried by the subject (e.g. the apparatus 4 is portable), for example a wearable device, such as part of a pendant or arm/wrist band or a smartphone. In alternative embodiments, the apparatus 4 is in or part of an electronic device such as a laptop, desktop computer or server that can be in the environment of interest (e.g. in a network gateway in a house), or remote from the environment of interest.

The first movement sensor 6 and at least one motion sensor 8 provide measurements of movements and motion respectively to the apparatus 4 for processing. The sensors 6, 8 can be connected using wires to the apparatus 4 or connected wirelessly using any suitable communication technology, for example Wi-Fi, Bluetooth, Bluetooth low energy (BTLE), cellular communications, ZWave, Zigbee, etc. The mode of connection and communication between the sensors 6, 8, 10, 12 and the apparatus 4 can depend on the form of the apparatus 4. In some embodiments, the apparatus 4 can comprise one or more inputs, e.g. connectors, ports, etc., through or via which the measurements from the first movement sensor 6 and/or motion sensor(s) 8, 10, 12 can be provided to the apparatus 4 for analysis or processing. In this case, the first movement sensor 6 and/or motion sensor(s) 8, 10, 12 can be connected or coupled to the input.

For example, where the apparatus 4 is located in the environment of interest, the at least one motion sensor 8 can be connected to the apparatus 4 using a wired connection (since the motion sensor 8 is also typically in a fixed location in the environment), and the first movement sensor 6 can be connected to the apparatus 4 wirelessly (since the first movement sensor 6 is typically worn or carried by the subject). Alternatively, where the apparatus 4 is in a form in which it is worn or carried by the subject, such as a smartphone, the first movement sensor 6 can be connected to the apparatus 4 using a wired or wireless connection, e.g. if the movement sensor 6 is worn on the clothing, wrist or around the neck in the form of a pendant, (or perhaps the first movement sensor 6 can be part of the same device as the apparatus 4), and the at least one motion sensor 8 can be connected to the apparatus 4 using a wireless connection.

The first movement sensor 6 and/or the at least one motion sensor 8 can provide their measurements to the apparatus 4 is real-time or near real-time, or alternatively the first movement sensor 6 and/or the at least one motion sensor 8 can store measurements locally (i.e. in the sensor 6, 8) and provide (communicate) the measurements to the apparatus 4 as required, for example in response to a request for the measurements from the apparatus 4.

The apparatus 4 comprises a processing unit 14 that obtains the measurements of movement from the first movement sensor 6 and measurements of motion from the first motion sensor 8 (and measurements of motion from the second motion sensor 10 and third motion sensor 12 if present in the system 2), and processes or analyses the measurements according to the invention in order to monitor the subject of interest. Where the apparatus 4 obtains the measurements in real-time or near real-time, the apparatus 4 can process the measurements in real-time or near real-time, or alternatively the apparatus 4 can store the measurements for processing at a later stage (for example at the end of each hour, day, or other monitoring period). In addition, the processing unit 14 can control the operation of the apparatus 4, or more generally the operation of the system 2, for example activating and/or deactivating the one or more of the sensors 6, 8, 10, 12 as required.

The processing unit 14 can be implemented in numerous ways, with software and/or hardware, to perform the various functions described below. The processing unit 14 may comprise one or more microprocessors or digital signal processor (DSPs) that may be programmed using software or computer program code to perform the required functions and/or to control components of the processing unit 14 to effect the required functions. The processing unit 14 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

In various implementations, the processing unit 14 may be associated with or comprise one or more memory units 16 such as volatile and non-volatile computer memory such as RAM, PROM, EPROM, and EEPROM. The processing unit 14 or associated memory unit 16 can also be used for storing program code that can be executed by a processor in the processing unit 14 to perform the method described herein. The memory unit 16 can also be used to store signals or measurements received from the first movement sensor 6 and measurements of motion from the first motion sensor 8 (and measurements of motion from the second motion sensor 10 and third motion sensor 12 if present in the system 2). This is particularly useful where the measurements from the sensors 6, 8, 10, 12 are stored and processed at a later stage.

In some embodiments, which are described in more detail below, the system 2 can also comprise a second movement sensor 18. This movement sensor 18 is for measuring movements of another person (i.e. not the subject of interest) or an animal that may be in the environment of interest. The details of the second movement sensor 18 can correspond to the details of the first movement sensor 6 described above.

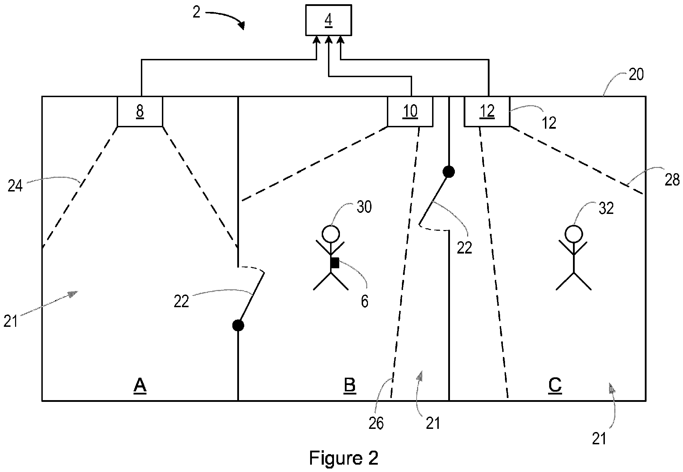

Figure 2 shows a system 2 according to an embodiment in use in an environment of interest. In particular, Figure 2 shows an environment of interest 20 that comprises three rooms 21, labelled as room A, room B and room C. Room A and room B are connected via a door 22, and room B and room C are connected via another door 22. The first motion sensor 8 in the system 2 is located in room A and measures motion in a sensing region, which is generally indicated by dashed lines 24. The second motion sensor 10 is located in room B and measures motion in a sensing region, which is generally indicated by dashed lines 26. The third motion sensor 12 is located in room C and measures motion in a sensing region, which is generally indicated by dashed lines 28. The motion sensors 8, 10, 12 in this embodiment are PIR sensors, but it will be appreciated that other types of motion sensors 8, 10, 12 can be used, such as open-close sensors, power sensors, or carbon dioxide sensors. Each of the first motion sensor 8, second motion sensor 10 and third motion sensor 12 are connected to apparatus 4. A subject of interest 30 (hereinafter referred to simply as 'the subject') is wearing the first movement sensor 6. In Figure 2 the subject 30 is located in room B within the sensing region of the second motion sensor 10. Another person 32 is shown in room C. The motion sensors 8, 10, 12 can be mounted or attached to the walls or ceilings in the rooms 21.

As described above, the apparatus 4 obtains the measurements from the movement sensor 6 and motion sensor(s) 8 in the environment and compares the measurements to determine if motion measured by a motion sensor 8 is motion by the subject 30. The apparatus 4 aims to determine a correspondence or correlation between the movements measured by the first movement sensor 6 and motion measured by the (e.g. wall-mounted) motion sensor(s) 8 to identify which motion is of interest (i.e. relating to the subject 30).

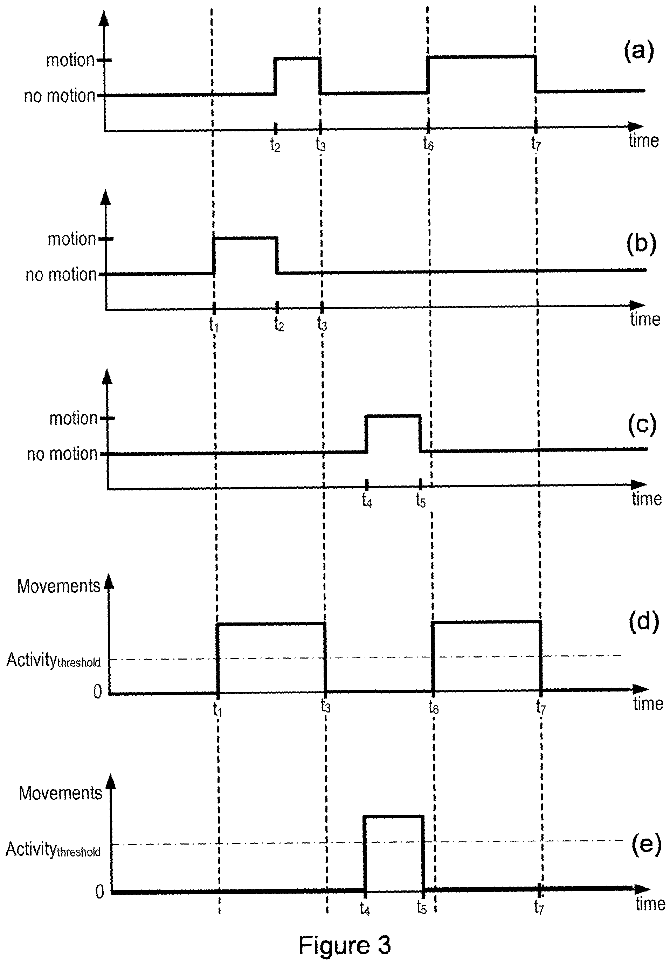

The graphs in Figure 3 illustrate an exemplary set of measurements from the sensors 6, 8, 10, 12 in the system 2 of Figure 2. In particular, Figure 3(a) shows measurements from the first motion sensor 8, Figure 3(b) shows measurements from the second motion sensor 10, Figure 3(c) shows measurements from the third motion sensor 12 and Figure 3(d) shows a simplified indication of measurements of movement of the subject 30 from the first movement sensor 6. These measurements are all provided to the apparatus 4 for processing.

It can be seen that the second motion sensor 10 in room B, where the subject 30 is located in Figure 2, measures motion in a time period starting at time t₁ and ending at time t₂. The first motion sensor 8 in room A measures motion in two time periods, a time period starting at time t₂ and ending at time t₃ a subsequent time period starting at time t₆ and ending at time t₇. The third motion sensor 12 in room C measures motion in a time period starting at time t₄ and ending at time t₅. It will be noted that this time period is between the two time periods in which motion is detected by the first motion sensor 8. The measurements by the first movement sensor 6, which are shown in a simplified binary form in Figure 3(d) with reference to a threshold amount (indicated by the dashed line labelled Activity_{threshold}) show that the subject 30 was moving in a time period starting at time t₁ and ending at time t₃ and a subsequent time period starting at time t₆ and ending at time t₇. It will be appreciated that the binary signal shown in Figure 3(d) can be obtained by, for example, determining the variance in the norm of the movement measurements over a time window (e.g. 1 second in length), and comparing the variance to the threshold. In some embodiments, the value of the threshold can be adapted dynamically so as to maximise the correlation with measurements from one of the motion sensors 8, 10, 12 while minimising the correlation with the measurements from the other motion sensors 8, 10, 12.

Thus, based on the measurements shown in Figure 3, the apparatus 4 needs to determine whether any of the periods of detected motion are due to movements of the subject 30 (and therefore whether the subject 30 is located in any of the sensing regions of the motion sensors 8, 10, 12 i.e. whether the subject 30 is in room A, room B or room C). From a comparison of the motion measurements and the movement measurements, it can be seen that the subject 30 is moving in time period t₁ to t₃, which corresponds to (i.e. occurs at the same time as) the motion detected in room B by the second motion sensor 10 in the time period t₁ to t₂ and the motion detected in room A by the first motion detector 8 in the time period t₂ to t₃. Consequently, the apparatus 4 can determine that the motion measured by the second motion sensor 10 in time period t₁ to t₂ and the motion measured by the first motion sensor 8 in time period t₂ to t₃ are due to motion of the subject 30 (for example due to the subject 30 moving from room B to room A).

A comparison of the measurements also shows that the subject 30 is not moving in the time period starting at time t₄ and ending at time t₅ when the third motion sensor 12 measures motion in room C, so the apparatus 4 can determine that this motion is not motion by the subject 30.

However, the motion measured by the first motion sensor 8 in room A in the time period t₆ to t₇ corresponds with movements measured by the first movement sensor 6, and so the apparatus 4 can determine that the motion in room A in this time period is due to the subject 30.

As noted above, in some embodiments the system 2 can comprise additional movement sensors that are to be worn or carried by other people or animals. For example, person 32 shown in Figure 2 in room C can wear or carry the second movement sensor 18. Figure 3(e) shows measurements of movement of the person 32 from the second movement sensor 18 in a simplified binary form with reference to a threshold amount. It will be appreciated that the value of the Activity_{threshold} can be the same as or different to that used to evaluate the measurements by the first movement sensor 6.

In the same way that the apparatus 4 can compare the measurements of the movement by the subject 30 from the first movement sensor 6 to the measurements of motion to determine whether the motion is due to the subject 30, the apparatus 4 can compare the measurements of the movement by the person 32 from the second movement sensor 18 to the measurements of motion to determine whether the motion is due to the person 32.

Thus, as shown in Figure 3(e) the person 32 is not moving in the time periods t₁ to t₃ or t₆ to t₇, and thus the motion measured in these time periods is not due to motion by the person 32. However, the movement measurements show that the person 32 is moving in the time period t₄ to t₅, and thus the apparatus 4 can determine that the motion measured by the third motion sensor 12 in this time period is due to movements by the person 32 rather than the subject 30.

More generally, the apparatus 4 can be configured so that if the measurements from the first movement sensor 6 indicate that the subject 30 is not moving, any motion detected by the motion sensor(s) 8, 10, 12 at that time can be discarded as not relating to the subject 30. In an alternative configuration that can be useful where, for example, a person lives alone with a pet and the movements/location of the person around the home are to be monitored, the pet can wear the first movement sensor 6 instead of the person (which is more convenient for the person). Thus, in this case if the measurements from the first movement sensor 6 indicate that the subject 30 (i.e. the pet) is moving at the same time as motion is detected by a motion sensor 8, 10, 12, this part of the motion sensor signal can be discarded as it relates to movements of the pet (subject 30) rather than the person. Since the person lives alone, it can therefore be assumed that other motion detected by the motion sensors 8, 10, 12 that coincides with no measured movement by the pet 30 is due to the person.

In some embodiments, the apparatus 4 can determine from the measurements from the first movement sensor 6 whether the first movement sensor 6 is being worn or carried by the subject 30. In that case, if the apparatus 4 determines that the first movement sensor 6 is not being worn or carried by the subject 30, the apparatus 4 can ignore the measurements from the first movement sensor 6 and just process the measurements of motion to determine the location of the subject.

In some embodiments, when it has been determined that the subject 30 is in a particular location or room (e.g. the subject 30 is in room A after time t₃), any motion detected by the second motion sensor 10 in room B or motion detected by the third motion sensor 12 in room C can be discarded or assigned to another person 32 or a pet.

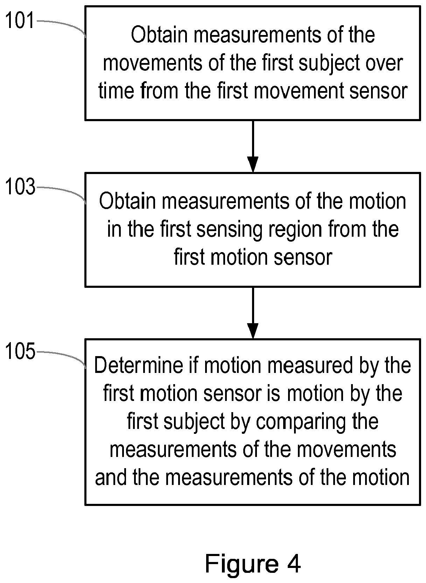

The flow chart in Figure 4 shows a method of monitoring of subject 30 according to an embodiment. The method can be performed by apparatus 4, and specifically by processing unit 14. The subject 30 has the first movement sensor 6 for measuring the movements of the subject 30, and the first motion sensor 8 is located in the environment of interest 20, with the first motion sensor 8 measuring motion in a first sensing region 24.

In a first step, step 101, the apparatus 4 obtains measurements of the movements of the subject 30 over time from the first movement sensor 6. Step 101 can comprise obtaining (e.g. receiving) the measurements directly from the first movement sensor 6, or obtaining the measurements by retrieving the measurements of the movements from a memory unit 16.

In a second step, step 103, the apparatus 4 obtains measurements of the motion in the first sensing region from the first motion sensor 8. Step 103 can comprise obtaining (e.g. receiving) the measurements directly from the first motion sensor 8, or obtaining the measurements by retrieving the measurements of the motion from a memory unit 16. It will be appreciated that the measurements of the movements and measurements of the motion cover the same time period.

Next, in step 105, the apparatus 4 determines if motion measured by the first motion sensor 8 is motion by the subject 30 (and thus whether the subject 30 is in the first sensing region 24) by comparing the measurements of the movements and the measurements of the motion.

In some embodiments, step 105 can comprise determining that motion measured by the first motion sensor 8 is motion by the subject 30 and that the subject 30 is in the first sensing region 24 if the motion measured by the first motion sensor 8 occurs during a time period in which the measurements of the movements indicate that the subject 30 is moving. Thus, in the example of Figure 3, step 105 can comprise determining that motion measured in the time period t₂ to t₃ in the first sensing region 24 is motion by the subject 30 as the subject 30 is moving in that time period according to the measurements by the first movement sensor 6.

In some embodiments, step 105 can comprise determining that motion measured by the first motion sensor 8 is not motion by the subject 30 if the motion measured by the first motion sensor 8 occurs during a time period in which the measurements of the movements indicate that the subject 30 is not moving. This would correspond to, for example, the time period t₄ to t₅ in the case of the third motion sensor 12.

In some embodiments, the environment of interest 20 comprises a second motion sensor 10 that is for measuring motion in a second sensing region 26 (e.g. room B), and the method can further comprise obtaining measurements of the motion in the second sensing region 26 from the second motion sensor 10 (relating to the same time period as the measurements from the first movement sensor 6 and first motion sensor 8) and step 105 can comprise determining if motion measured by the first motion sensor 8 and/or motion measured by the second motion sensor 10 is motion by the subject 30 by comparing the measurements of the movements, the measurements of the motion from the first motion sensor 8 and the measurements of the motion from the second motion sensor 10.

This comparison of the measurements can comprise determining that motion measured by the first motion sensor 8 is motion by the subject 30 if the motion measured by the first motion sensor 8 occurs during a time period in which the measurements of the movements indicate that the subject 30 is moving. The comparison can also comprise determining that motion measured by the second motion sensor 10 is motion by the subject 30 if the motion measured by the second motion sensor 10 occurs during a time period in which the measurements of the movements indicate that the subject 30 is moving.

It will be appreciated that in this embodiment it may not be possible to immediately distinguish the location of the subject 30 if both the first motion sensor 8 and the second motion sensor 10 detect motion at the same time as the subject 30 is moving (e.g. if the subject 30 is moving near the first motion sensor 8 and a cat is moving at the same time near the second motion sensor 10). However, the apparatus 4 can correlate the measurements from the first movement sensor 6 with the measurements from both motion sensors 8, 10 and determine which motion sensor 8, 10 the movements correlate with the best. In further embodiments, the apparatus 4 can analyse movement-motion pairs and historical (including recent) patterns to determine the most likely location (sensing region) for the subject 30.

In some embodiments, another person 32 (also referred to as a "second subject") has a second movement sensor 18 for measuring the movement of the person 32, and the method further comprises the step of obtaining measurements of the movements of the person 32 from the second movement sensor 18 (relating to the same time period as the measurements from the first movement sensor 6 and first motion sensor 8) and step 105 can comprise determining if motion measured by the first motion sensor 8 is motion by the subject 30 or the person 32 by comparing the measurements of the movements of the subject 30, the measurements of the movements of the person 32 and the measurements of the motion.

This comparison of the measurements can comprise determining that motion measured by the first motion sensor 8 is motion by the subject 30 if the motion measured by the first motion sensor 8 occurs during a time period in which the measurements of the movements indicate that the subject 30 is moving. This comparison can also comprise determining that motion measured by the first motion sensor 8 is motion by the person 32 if the motion measured by the first motion sensor 8 occurs during a time period in which the measurements of the movements indicate that the person 32 is moving.

In some embodiments, step 105 can comprise correlating the measurements of the movements of the subject 30 with measurements of motion from each of the motion sensors 8, 10, 12 in the system 2 and identifying the motion sensor(s) that provide the highest correlation (in which case the motion measured by that motion sensor or those motion sensors can be determined to be motion by the subject 30). In some cases the time window over which correlation is determined is varied to allow for the subject 30 to move between rooms (and thus move between motion sensors 8, 10, 12).

Thus, the invention provides that using a movement sensor to measure the movements of a subject enables false-positive motion signals from a motion sensor to be discarded or disregard, but also enables tracking of the location of the subject more accurate as more specific information is gathered about the movement/activity of the subject in addition to the presence in a certain room, location or sensing region.

There is therefore provided an improved method, apparatus and system for monitoring a subject in an environment of interest.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method for monitoring a first subject in an environment of interest using a first movement sensor for measuring movements of the first subject, and a first motion sensor in the environment of interest for measuring motion in a first sensing region, the method comprising:
obtaining measurements of the movements of the first subject over time from the first movement sensor;
obtaining measurements of the motion in the first sensing region from the first motion sensor; and
determining if motion measured by the first motion sensor is motion by the first subject by comparing the measurements of the movements and the measurements of the motion.

2. A method as claimed in claim 1, wherein the step of determining comprises determining that motion measured by the first motion sensor is motion by the first subject and that the first subject is in the first sensing region if the motion measured by the first motion sensor occurs during a time period in which the measurements of the movements indicate that the first subject is moving.

3. A method as claimed in claim 1 or 2, wherein the step of determining comprises determining that motion measured by the first motion sensor is not motion by the first subject if the motion measured by the first motion sensor occurs during a time period in which the measurements of the movements indicate that the first subject is not moving.

4. A method as claimed in claim 1, 2 or 3, wherein the method further comprises the step of:
obtaining measurements of motion in a second sensing region from a second motion sensor; and
wherein the step of determining comprises determining if motion measured by the first motion sensor and/or motion measured by the second motion sensor is motion by the first subject by comparing the measurements of the movements, the measurements of the motion from the first motion sensor and the measurements of the motion from the second motion sensor.

5. A method as claimed in claim 4, wherein the step of determining if motion measured by the first motion sensor and/or motion measured by the second motion sensor is motion by the first subject comprises:
determining that motion measured by the first motion sensor is motion by the first subject if the motion measured by the first motion sensor occurs during a time period in which the measurements of the movements indicate that the first subject is moving; and/or
determining that motion measured by the second motion sensor is motion by the first subject if the motion measured by the second motion sensor occurs during a time period in which the measurements of the movements indicate that the first subject is moving.

6. A method as claimed in any of claims 1-5, wherein a second subject has a second movement sensor for measuring the movement of the second subject, and the method further comprises the step of:
obtaining measurements of the movements of the second subject from the second movement sensor; and
wherein the step of determining comprises determining if motion measured by the first motion sensor is motion by the first subject or the second subject by comparing the measurements of the movements of the first subject, the measurements of the movements of the second subject and the measurements of the motion.

7. A method as claimed in claim 6, wherein the step of determining if motion measured by the first motion sensor is motion by the first subject or the second subject comprises:
determining that motion measured by the first motion sensor is motion by the first subject if the motion measured by the first motion sensor occurs during a time period in which the measurements of the movements indicate that the first subject is moving; and
determining that motion measured by the first motion sensor is motion by the second subject if the motion measured by the first motion sensor occurs during a time period in which the measurements of the movements indicate that the second subject is moving.

8. A computer program product comprising a computer readable medium having computer readable code embodied therein, the computer readable code being configured such that, on execution by a suitable computer or processor, the computer or processor is caused to perform the method of any of claims 1-7.

9. An apparatus for monitoring a first subject in an environment of interest, the apparatus comprising:
a processing unit configured to:
obtain measurements of movements of the first subject over time from a first movement sensor of the first subject;
obtain measurements of motion in a first sensing region from a first motion sensor in the environment of interest; and
determine if motion measured by the first motion sensor is motion by the first subject by comparing the measurements of the movements and the measurements of the motion.

10. An apparatus as claimed in claim 9, wherein the processing unit is configured to determine that motion measured by the first motion sensor is motion by the first subject and that the first subject is in the first sensing region if the motion measured by the first motion sensor occurs during a time period in which the measurements of the movements indicate that the first subject is moving.

11. An apparatus as claimed in claim 9 or 10, wherein the processing unit is configured to determine that motion measured by the first motion sensor is not motion by the first subject if the motion measured by the first motion sensor occurs during a time period in which the measurements of the movements indicate that the first subject is not moving.

12. An apparatus as claimed in claim 9, 10 or 11, wherein the processing unit is further configured to:
obtain measurements of motion in a second sensing region from a second motion sensor in the environment of interest; and
wherein the processing unit is configured to determine if motion measured by the first motion sensor and/or motion measured by the second motion sensor is motion by the first subject by comparing the measurements of the movements, the measurements of the motion from the first motion sensor and the measurements of the motion from the second motion sensor.

13. An apparatus as claimed in claim 12, wherein the processing unit is configured to determine that motion measured by the first motion sensor is motion by the first subject if the motion measured by the first motion sensor occurs during a time period in which the measurements of the movements indicate that the first subject is moving; and to determine that motion measured by the second motion sensor is motion by the first subject if the motion measured by the second motion sensor occurs during a time period in which the measurements of the movements indicate that the first subject is moving.

14. An apparatus as claimed in any of claims 9-13, wherein the processing unit is further configured to
obtain measurements of movements of a second subject from a second movement sensor; and
wherein the processing unit is configured to determine if motion measured by the first motion sensor is motion by the first subject or the second subject by comparing the measurements of the movements of the first subject, the measurements of the movements of the second subject and the measurements of the motion.

15. A system for monitoring a first subject in an environment of interest, the system comprising:
an apparatus as claimed in any of claims 9-14;
a first movement sensor for measuring the movements of the first subject; and
an input for receiving measurements of motion in a first sensing region from a first motion sensor in the environment of interest.
